# EUROPÄISCHE PATENTANMELDUNG

(11) **EP 1 340 742 A1**
(43) Veröffentlichungstag der Anmeldung: **03.09.2003**
(21) Anmeldenummer: 03002154.7
(22) Anmeldetag: 03.02.2003
(51) Int. Cl.: C07C 201/08, C07C 205/12

(54) **Kontinuierliches adiabatisches Verfahren zur Nitrierung von Chlorbenzol**

(30) Priorität: 13.02.2002 DE 10205855
(71) Anmelder: Bayer Aktiengesellschaft, 51368 Leverkusen (DE)
(72) Erfinder: Demuth, Ralf, Dr., 40724 Hilden (DE); Gotta, Matthias, Dr., 51061 Köln (DE); Linn, Thomas, Dr., 41517 Grevenbroich (DE); Weber, Martin, Dr., 51373 Leverkusen (DE); Zierngiebl, Eberhard, Dr., 51061 Köln (DE)

(57) **Zusammenfassung**

Die vorliegende Erfindung betrifft ein kontinuierliches adiabatisches Verfahren zur Herstellung von Nitrochlorbenzol in Gegenwart von Phosphorsäure. Die bei der Durchführung des Verfahrens anfallende Abfallsäure wird aufkonzentriert und in die Nitrierreaktion zurückgeführt.

## Beschreibung

Die vorliegende Erfindung betrifft ein kontinuierliches adiabatisches Verfahren zur Herstellung von Nitrochlorbenzol, wobei die bei der Durchführung des Verfahrens anfallende Abfallsäure aufkonzentriert und in die Nitrierreaktion zurückgeführt wird. Durch Zusatz von Phosphorsäure kann der Anteil des Wertproduktes ortho-Nitrochlorbenzol signifikant gesteigert werden. Die Produktion des Koppelproduktes para-Nitrochlorbenzol wird zugunsten des Wertproduktes zurückgedrängt.

Nitrochlorbenzole sind wichtige Zwischenprodukte zur Herstellung von Farbstoffen, Pharmazeutika und Schädlingsbekämpfungsmitteln. Besonders große Nachfrage besteht nach dem bei der herkömmlichen Nitrierung von Chlorbenzol zu ca. 35 % anfallenden ortho-Nitrochlorbenzol. Auch für para-Nitrochlorbenzol gibt es einige technische Verwendungen. Da für meta-Nitrochlorbenzol, nur sehr eingeschränkte technische Verwendungsmöglichkeiten bestehen, ist dessen Anfall in der Regel unerwünscht.

Nitrochlorbenzole werden technisch durch Nitrierung von Chlorbenzol hergestellt. Zur Nitrierung wird üblicherweise eine Mischung aus Schwefelsäure, Salpetersäure und Wasser eingesetzt. Unter diesen Bedingungen ist der Anteil des para-Isomeren sehr hoch. Das Verhältnis von para- zu ortho-Nitrochlorbenzol beträgt üblicherweise etwa 1,95. Zusätzlich fällt ein erheblicher Anteil mit organischen Verbindungen belasteter Abfallschwefelsäure an, der verfahrens- und kostenintensiv aufgearbeitet werden muss. Besonders elegant gelingt die Aufkonzentrierung der Abfallschwefelsäure bei Durchführung der Nitrierreaktion unter adiabatischen Bedingungen, da hierbei kein Wärmeaustausch mit der Umgebung erfolgt und die bei dem Verfahren frei werdende Energie zur Vorwärmung bzw. Aufkonzentrierung der Abfallschwefelsäure verwendet werden kann. Unter adiabatischen Bedingungen kann auch ein deutlich verbessertes para- zu ortho-Nitrochlorbenzol Verhältnis erzielt werden. Wird ein Teil der Schwefelsäure durch Phosphorsäure ersetzt, so kann ein deutlich verbessertes para- zu ortho-Nitrochlorbenzol Verhältnis erzielt werden und die energetisch günstigen Bedingungen der Aufkonzentrierung der Abfallsäure können beibehalten werden.

Der Einfluss von Phosphorverbindungen auf Nitrierungen ist in der Literatur bekannt. So beschreibt DE 2 422 305 den Zusatz von Phosphorsäure bei der isothermen Nitrierung von Chlorbenzol und die damit verbundene größere Ausbeute an ortho-Nitrochlorbenzol. Dabei geht generell der Einsatz von Phosphorsäure mit einer Erniedrigung der Reaktionsgeschwindigkeit einher. Zur Erzielung wirtschaftlicher Raum-Zeit-Ausbeuten muss auf Phosphorsäuren hoher Konzentration zurückgegriffen werden (>100 % H₃PO₄). Die Aufkonzentrierung und Rückführung dieser Phosphorsäuren ist allerdings sehr aufwändig. Alternativ dazu kann die Nitrierung auch bei einer erhöhten Reaktionstemperatur durchgeführt werden. Dies führt allerdings u.a. zu einem erhöhten Anteil an unerwünschtem m-Nitrochlorbenzol, welches unter hohem Aufwand abgetrennt werden muss.

In US 4,453,027 wird ein adiabatisches Nitrierverfahren zur Herstellung von Mononitrohalogenbenzolen beansprucht. Die verwendete Nitriersäure enthält aber so große Mengen Salpetersäure, dass bei der beschriebenen Endtemperatur von 100 bis 110°C die Starttemperatur der Reaktion und folglich auch der Reaktanden bei 0 bis 10°C liegen muss. Ein solches Verfahren ist technisch und wirtschaftlich nicht sinnvoll, da Kühlsole und kostenintensive Apparaturen eingesetzt werden müssen.

In EP 675 104 A ist ein adiabatisches Verfahren zur Nitrierung von Halogenbenzolen beschrieben, wobei die Reaktanden unter Aufwand einer bestimmten Mischenergie vermischt werden und die Vermischung im Temperaturbereich von 60 bis 160°C erfolgt. Mit den genannten Temperaturen lassen sich die für eine adiabatische Fahrweise notwendigen hohen Reaktionsgeschwindigkeiten erzielen. Nachteilig an diesem Verfahren ist aber, dass relativ hohe Anteile an unerwünschtem meta-Nitrochlorbenzol erhalten werden, deren Abtrennung, wie bereits erwähnt, aufwendig und kostenintensiv ist.

Es bestand daher Bedarf an einem Verfahren zur kontinuierlichen Herstellung von Nitrochlorbenzol, welches eine einfache Aufarbeitung und Rückführung der Abfallsäure erlaubt und eine deutlich gesteigerte Ausbeute des Zielproduktes ortho-Nitrochlorbenzol aufweist und das unerwünschte Nebenprodukt meta-Nitrochlorbenzol möglichst vermeidet.

Überraschenderweise wurde nun ein Verfahren zur kontinuierlichen Herstellung von Nitrochlorbenzol durch Umsetzung von Chlorbenzol mit Schwefelsäure, Phosphorsäure, Salpetersäure und Wasser gefunden, das dadurch gekennzeichnet ist, dass
a) die Einsatzstoffe Chlorbenzol, Schwefelsäure, Phosphorsäure, Salpetersäure und Wasser gleichzeitig oder in beliebiger Reihenfolge sukzessive in einen mit Mischungsorganen ausgestatteten Reaktor eingetragen werden und die Temperatur des Reaktionsgemisches bei der Erstvermischung 10 bis 80°C beträgt,
b) der Gehalt an Phosphorsäure im Reaktionsgemisch bei der Vermischung, bezogen auf die Summe aus Phosphorsäure, Schwefelsäure, Salpetersäure und Wasser, 10 bis 50 Gew.-% beträgt,
c) die Reaktion unter adiabatischen Bedingungen abläuft,
d) am Reaktorausgang das rohe Nitrochlorbenzol von der Abfallsäure abgetrennt wird und
e) die Abfallsäure aufkonzentriert wird und als recycliertes Säuregemisch wieder in die Nitrierreaktion zurückgeführt wird und
f) gegebenenfalls bis zu 3000 ppm einer Siliziumverbindung zugesetzt werden können.

Im erfindungsgemäßen Verfahren werden als Einsatzstoffe, Chlorbenzol, Schwefelsäure, Phosphorsäure, Salpetersäure und Wasser eingesetzt, wobei das eingesetzte Chlorbenzol Nitrochlorbenzol in einer Menge von beispielsweise 0 bis 20 Gew.-% enthalten kann. Ein höhere Menge ist möglich, aber unwirtschaftlich. Wasser kann dabei als solches eingesetzt werden oder aber als Verdünnungswasser in der Salpetersäure und/oder der Schwefelsäure und/oder der Phosphorsäure in die Reaktion eingebracht werden.

Die Einsatzstoffe Chlorbenzol, Schwefelsäure, Phosphorsäure, Salpetersäure und Wasser können im erfindungsgemäßen Verfahren einzeln oder als Mischungen in einen mit Mischungsorganen ausgestatteten Reaktor eingetragen werden. Der Eintrag der Einsatzstoffe in den Reaktor kann gleichzeitig oder in beliebiger Reihenfolge sukzessive erfolgen. Der Eintrag in den Reaktor kann beispielsweise so erfolgen, dass Chlorbenzol und Salpetersäure und gegebenenfalls Wasser als separate Ströme gleichzeitig oder sukzessive dem recyclisierten Säuregemisch zugesetzt werden, wobei die Salpetersäure durch Wasser verdünnt sein kann. Chlorbenzol kann auch mit Wasser und Schwefelsäure und/oder Phosphorsäure vorgemischt werden und die resultierende Mischung als separater Strom in den Reaktor eingetragen werden. Dort erfolgt eine Vermischung mit Salpetersäure, welche mit Schwefelsäure und/oder Phosphorsäure und/oder Wasser vermischt sein kann. Weiterhin können Chlorbenzol und eine Nitriersäure, welche durch Mischen von Schwefelsäure, Phosphorsäure, Salpetersäure und gegebenenfalls Wasser hergestellt wurde, in separaten Strömen in den Reaktor eingetragen werden. In einer bevorzugten Form des erfindungsgemäßen Verfahrens werden Salpetersäure und das recyclisierte Säuregemisch zu einer Nitriersäure vermischt und die Nitriersäure sowie Chlorbenzol in separaten Strömen in den Reaktor eingetragen. Für das Gelingen der Reaktion ist es unerheblich, in welcher Reihenfolge und Zusammensetzung die Reaktanden in den Reaktor eingetragen werden, solange das Reaktionsgemisch, welches sich nach Vermischung aller Reaktanden ergibt die erfindungsgemäße Zusammensetzung aufweist und bei der erfindungsgemäßen Temperatur stattfindet.

Der Gehalt an Phosphorsäure bezogen auf reine H₃PO₄ im Reaktionsgemisch zum Zeitpunkt der Vermischung kann, bezogen auf die Summe von Schwefelsäure bezogen auf reine H₂SO₄, Phosphorsäure bezogen auf reine H₃PO₄, Salpetersäure bezogen auf reine HNO₃ und Wasser, 10 bis 50 Gew.-%, bevorzugt 15 bis 50 Gew.-% besonders bevorzugt 20 bis 45 Gew.-% betragen.

Der Gehalt an Schwefelsäure kann, bezogen auf reine H₂SO₄ im Reaktionsgemisch zum Zeitpunkt der Vermischung, bezogen auf die Summe von Schwefelsäure bezogen auf reine H₂SO₄, Phosphorsäure bezogen auf reine H₃PO₄, Salpetersäure bezogen auf reine HNO₃ und Wasser, 30 bis 80 Gew.-%, bevorzugt 40 bis 75 Gew.-%, besonders bevorzugt 40 bis 65 Gew.-% betragen. Schwefelsäure kann beispielsweise mit einem Gehalt von 65 bis 100 Gew.-% Schwefelsäure, bevorzugt 80 bis 100 Gew.-% eingesetzt werden.

Der Gehalt an Salpetersäure, bezogen auf reine HNO₃ zum Zeitpunkt der Vermischung kann, bezogen auf die Summe von Schwefelsäure bezogen auf reine H₂SO₄, Phosphorsäure bezogen auf reine H₃PO₄, Salpetersäure bezogen auf reine HNO₃ und Wasser, 3 bis 10 Gew.-%, bevorzugt 4 bis 8 Gew.-%, besonders bevorzugt 4 bis 6 Gew.-% betragen. Salpetersäure kann beispielsweise mit einem Gehalt von 60 bis 98 Gew.-% Salpetersäure, bevorzugt aber in Form der ca. 60 bis 70 gew.-%igen konzentrierten Salpetersäure eingesetzt werden.

Der Gehalt an Wasser kann im Reaktionsgemisch zum Zeitpunkt der Vermischung, bezogen auf die Summe von Schwefelsäure bezogen auf reine H₂SO₄, Phosphorsäure bezogen auf reine H₃PO₄, Salpetersäure bezogen auf reine HNO₃ und Wasser, 5 bis 30 Gew.-%, bevorzugt 5 bis 20 Gew.-%, besonders bevorzugt 8 bis 18 Gew.-% betragen.

Pro Mol Chlorbenzol können beispielsweise 0,5 bis 2 Mol Salpetersäure, bezogen auf reine HNO₃ eingesetzt werden, bevorzugt 1,0 bis 1,3, besonders bevorzugt 1,0 bis 1,2 und ganz besonders bevorzugt 1,05 bis 1,1 Mol.

In einer bevorzugten Ausführungsform des erfindungsgemäßen Verfahrens, werden die Reaktanden so vermischt, dass im Reaktor eine mittlere Mischleistungsdichte von 1,5 bis 40 Watt/l, bevorzugt 1,5 bis 30 Watt/l, vorliegt. Zur Vermischung können die in der Technik bekannten Mischorgane wie beispielsweise statische Mischer, Pumpen, Düsen, Rührer oder Kombinationen hiervon eingesetzt werden. Die Bestimmung der Mischleistungsdichte, ausgedrückt in Watt pro Liter, in einem kontinuierlich betriebenen Reaktor wird folgendermaßen durchgeführt:
Mischleistungsdichte = Leistung P / Volumen V [W/1]
P = Durchsatz der Reaktanden [m³/s] x dynamischem Druckabfall ΔP_{dyn} [N/m²]
ΔP_{dyn} = gesamter Druckabfall ΔP_{ges} - statischer Druckabfall ΔPₛₜₐₜ

Da die mittlere Mischleistungsdichte auf jeden Liter der Reaktionsmischung einwirkt und diese Reaktionsmischung erst im Reaktor vorliegt, wird bei der Berechnung der mittleren Mischleistungsdichte als Volumen V das Volumen des Reaktors eingesetzt, in welchem die Reaktion durchgeführt wird.

Sind Teile des Reaktors oder seiner Bestandteile aus Email oder kommt die Reaktionsmischung mit emaillierten Teilen in Kontakt, so ist es vorteilhaft, Siliziumverbindungen zuzusetzen. Die Menge kann dabei beispielsweise bis zu 3000 ppm, bevorzugt zwischen 10 und 3000 ppm und besonders bevorzugt zwischen 50 und 300 ppm liegen, wobei sich die Menge auf Massen-ppm bezogen auf das Gewicht der Reaktionsmischung bezieht. Dieser Zusatz unterdrückt den Angriff von Phosphorsäure an Email zumindest größtenteils. Als besonders vorteilhaft erweisen sich hier die verschiedenen Modifikationen von Siliziumoxiden und Kieselsäuren wie z.B. Wasserglas, Fällungskieselsäure oder Aerosil.

Die Temperatur des Reaktionsgemisches kann bei der Erstvermischung beispielsweise und bevorzugt 10 bis 80°C, besonders bevorzugt 20 bis 50°C und ganz besonders bevorzugt 30 bis 45°C betragen. Die Endtemperatur, die im Wesentlichen von der Temperatur der Erstvermischung und dem Umsatz abhängt, übersteigt im Allgemeinen 130°C nicht und liegt vorzugsweise zwischen 80 und 100°C.

Vorzugsweise sollte die Reaktion möglichst frei von Rückvermischung ablaufen, was beispielsweise durch Dispergierung der Reaktionsmischung erreicht werden kann. Dies wird vorzugsweise durch im Reaktor für diesen Zweck bestimmte Einbauten oder Organe wie beispielsweise Lochbleche, Schlitzbleche, Prallbleche, Strömungsbrecher, Umlenkbleche, Statikmischer oder Rührer bewerkstelligt.

Als kontinuierlich betriebene Reaktoren für das erfindungsgemäße Verfahren seien beispielsweise genannt: Rohrreaktoren, vorzugsweise mit Einbauten zur Dispergierung wie beispielsweise Lochbleche, Schlitzbleche, Prallbleche, Strömungsbrecher, Umlenkbleche, Statikmischer, Rührer und ähnliche, stark gerührte Kessel in Kaskadenanordnung, Schlaufenreaktoren mit wie oben beschriebenen Einbauten, Kombinationen mehrerer der genannten Apparate, weitere gleichwirkende Reaktoren, wie Kammerreaktoren mit Rührern in jeder Kammer. In bevorzugter Weise werden im erfindungsgemäßen Verfahren Rohrreaktoren mit Einbauten eingesetzt. Die Einbauten sind bevorzugt Lochbleche. Alle Einbauten stellen Unterteilungen der Gesamtapparatur dar, die gleichermaßen der Dispergierung und der weitgehenden Verhinderung der Rückvermischung dienen.

Nach dem intensiven Vermischen, nach jeder Dispergierung und nach dem Durchströmen einer gewissen Teillänge des Reaktors wird ein Koaleszieren der Dispersionströpfchen beobachtet, was durch Redispergierung rückgängig gemacht wird. Die Zahl der Dispergierungsvorgänge beträgt vorzugsweise 2 bis 50, bevorzugt 3 bis 30, besonders bevorzugt 4 bis 20. Die bei der Vermischung der Reaktanden auf jeden Liter des Reaktionsgemisches einwirkende mittlere Mischleistungsdichte von vorzugsweise 1,5 bis 40 Watt/l wird vorzugsweise zur Überwindung der bei den Dispergierungsvorgängen auftretenden Druckverluste eingesetzt.

Im erfindungsgemäßen Verfahren wird Chlorbenzol formelmäßig mit Salpetersäure zu Nitrochlorbenzol und Wasser umgesetzt. Somit werden Chlorbenzol und Salpetersäure in das Verfahren eingeführt und Nitrochlorbenzol und Wasser ausgeschleust, während das beschriebene Schwefelsäure/Phosphorsäure/Wassergemisch das Reaktionsmedium darstellt. Da bei der technischen Realisierung vorteilhafterweise wasserhaltige Salpetersäuren eingesetzt werden, muss zusätzlich zum Reaktionswasser auch noch das Wasser der eingesetzten wasserhaltigen Salpetersäure ausgeschleust werden.

Im erfindungsgemäßen Verfahren findet am Reaktorausgang eine Abtrennung des rohen Nitrochlorbenzols von der Abfallsäure statt. Die Abtrennung kann in dem Fachmann bekannten Apparaturen oder mit hinlänglich bekannten Mitteln erreicht werden. So kann eine Trennung beispielsweise durch Einsatz eines statischen Scheiders erfolgen. Die so erhaltene Abfallsäure ist weitgehend salpetersäurefrei und enthält zum überwiegenden Teil Schwefelsäure, Phosphorsäure und Wasser, gegebenenfalls sind zu einem geringen Anteil organische Verunreinigungen und/oder Nitrosylschwefelsäure enthalten. Die Schwefelsäure-, Phosphorsäure- und Wasserkonzentration der Abfallsäure ergeben sich durch die Eingangskonzentration und die Stöchiometrie der Reaktionsgleichung:

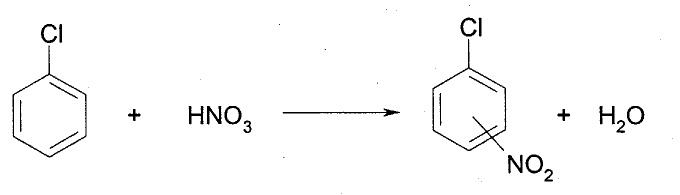

Zum Wiedereinsatz wird die Abfallsäure erfindungsgemäß aufkonzentriert und vorteilhafterweise in die Nitrierreaktion zurückgeführt. Bei der Aufkonzentrierung wird Wasser destillativ ausgeschleust. Hierzu wird in bevorzugter Weise die von der Abfallsäure aufgenommene Reaktionswärme ausgenutzt.

Die Aufkonzentrierung wird vorzugsweise in einem Verdampfer durchgeführt, welcher vorzugsweise bei einem Druck von 60 bis 200 mbar, besonders bevorzugt von 60 bis 180 mbar und ganz besonders bevorzugt von 80 bis 150 mbar betrieben wird. Die Temperatur der Abfallsäure im Austritt des Verdampfers beträgt dabei vorzugsweise 100 bis 200°C, besonders bevorzugt 130 bis 190°C und ganz besonders bevorzugt 145 bis 165°C. Die Temperatur der ablaufenden aufkonzentrierten Abfallsäure wird vorzugsweise dazu genutzt, um in einem Gegenstromwärmetauscher die in den Verdampfer strömende Abfallsäure aufzuheizen.

In einer bevorzugten Ausführungsform des erfindungsgemäßen Verfahrens wird durch die Aufkonzentrierung etwa so viel Wasser entfernt, wie in das Reaktionsmedium durch Reaktionswasser und gegebenenfalls wasserhaltige Salpetersäure gelangt ist.

Die Aufkonzentrierung wird vorzugsweise in einem einstufigen Verfahren durchgeführt, wobei vorzugsweise ein kommerziell erhältlicher, kaskadierter Verdampfer mit Tantalrohrbündel verwendet wird, bei dem vom Eintritt kommend mit jeder Kaskade die Säurekonzentration erhöht wird, so dass in den ersten Kaskaden eine relativ niederkonzentrierte Säure vorliegt. Vorteil der niedrigen Konzentration in der ersten Kaskade ist einerseits, dass der Siedepunkt noch niedrig ist und somit eine hohe treibende Temperaturdifferenz für den Wärmeübergang vorliegt und andererseits, dass bei höheren Wasserkonzentrationen evtl. in der Abfallsäure vorliegende Nitrosylschwefelsäure aus der Reaktion besser entfernbar ist. Somit wird durch die Verwendung eines kaskadierten Verdampfers im erfindungsgemäßen Verfahren das üblicherweise durchgeführte Ausblasen der Nitrosylschwefelsäure mit Schwefeldioxid und somit ein zusätzlicher Verfahrensschritt vermieden. Um Ablagerungen von organischen Verbindungen, insbesondere von Nitrochlorbenzol, auf dem Kondensator zu verhindern, wird dieses in einer bevorzugten Ausführungsform kontinuierlich mit Chlorbenzol berieselt. Die ablaufende organische Phase aus Chlorbenzol und Nitrochlorbenzol kann als Einsatzstoff im erfindungsgemäßen Verfahren eingesetzt werden.

Der Vorteil des erfindungsgemäßen Verfahren ist, dass durch den Einsatz eine Mischung von Phosphorsäure und Schwefelsäure als Reaktionsmedium eine erhöhte Ausbeute an ortho-Nitrochlorbenzol erreicht werden kann und eine Rückführung der Abfallsäure als recycliertes Säuregemisch verfahrenstechnisch günstig bewerkstelligt wird.

### Beispiele

### Beispiel 1

In einem wärmeisolierten Sulfierbecher (⌀ 100 mm), versehen mit Stromstören und zwei auf einer Welle sitzenden Turbinenrührern (⌀ 39,9 mm) wurden 418 g H₂SO₄ (100 %ig), 167 g Phosphorsäure (85 %ig), 61 g Wasser und 53,8 g HNO₃ (65 %ig) bei 40°C unter Rühren vorgelegt (eingebrachte spezifische Rührleistung 22 W/l) und in 3 Sekunden mit 68,7 g Chlorbenzol versetzt und ohne Kühlung zur Reaktion gebracht. Nach 120 Sekunden hatte das Reaktionsgemisch die Endtemperatur von 90°C erreicht und der Rührer wurde angehalten. Nach Phasentrennung wurden 85,3 g organische Phase erhalten.

| | |
|---|---|
| Chlorbenzol | 5,32 Gew.-% |
| ortho-Nitrochlorbenzol | 37,19 Gew.-% |
| meta-Nitrochlorbenzol | 0,75 Gew.-% |
| para-Nitrochlorbenzol | 56,25 Gew.-% |
| Dinitrochlorbenzole | 0,25 Gew.-% |

Das Verhältnis para/ortho-Nitrobenzol beträgt 1,50.

Die Abfallsäure wurde bei 110 mbar und 150°C wieder auf den ursprünglichen Wassergehalt aufkonzentriert und erneut mit Salpetersäure versetzt. Nach oben beschriebener Prozedur wurde eine erneute Nitrierung mit gleichen Resultaten durchgeführt. Auch nach mehrmaligen Zurückführen der Säure wurde kein Aktivitätsverlust beobachtet.

### Beispiel 2

In einem wärmeisolierten Sulfierbecher (⌀ 100 mm), versehen mit Stromstören und zwei auf einer Welle sitzenden Turbinenrührem (⌀ 39,9 mm) wurden 316,5 g H₂SO₄ (100 %ig), 323,0 g Phosphorsäure (85 %ig), 6,5 g Wasser und 53,8 g HNO₃ (65 %ig) bei 40°C unter Rühren vorgelegt (eingebrachte spezifische Rührleistung 22 W/l) und in 3 Sekunden mit 68,7 g Chlorbenzol versetzt und ohne Kühlung zur Reaktion gebracht. Nach 160 Sekunden hatte das Reaktionsgemisch die Endtemperatur von 90°C erreicht und der Rührer wurde angehalten. Nach Phasentrennung wurden 83,9 g organische Phase erhalten.

| | |
|---|---|
| Chlorbenzol | 5,59 Gew.-% |
| ortho-Nitrochlorbenzol | 38,30 Gew.-% |
| meta-Nitrochlorbenzol | 0,70 Gew.-% |
| para-Nitrochlorbenzol | 54,65 Gew.-% |
| Dinitrochlorbenzole | 0,23 Gew.-% |

Das Verhältnis para/ortho beträgt 1,41.

Die Abfallsäure wurde bei 110 mbar und 150°C wieder auf den ursprünglichen Wassergehalt aufkonzentriert und erneut mit Salpetersäure versetzt. Nach oben beschriebener Prozedur wurde eine erneute Nitrierung mit gleichen Resultaten durchgeführt. Auch nach mehrmaligen Zurückführen der Säure wurde kein Aktivitätsverlust beobachtet.

### Beispiel 3 (nicht erfindungsgemäß)

In einem wärmeisolierten Sulfierbecher (⌀ 100 mm), versehen mit Stromstören und zwei auf einer Welle sitzenden Turbinenrührern (⌀ 39,9 mm) wurden 646,2 g H₂SO₄ (81 %ig) und 53,8 g HNO₃ (65 %ig) bei 40°C unter Rühren vorgelegt (eingebrachte spezifische Rührleistung 22 W/l) und in 3 Sekunden mit 68,7 g Chlorbenzol versetzt und ohne Kühlung zur Reaktion gebracht. Nach 135 Sekunden hatte das Reaktionsgemisch die Endtemperatur von 94°C erreicht und der Rührer wurde angehalten. Nach Phasentrennung wurden 87,8 g organische Phase erhalten.

| | |
|---|---|
| Chlorbenzol | 6,25 Gew.-% |
| ortho-Nitrochlorbenzol | 34,43 Gew.-% |
| meta-Nitrochlorbenzol | 0,85 Gew.-% |
| para-Nitrochlorbenzol | 57,30 Gew.-% |
| Dinitrochlorbenzole | 0,14 Gew.-% |

Das Verhältnis para/ortho beträgt 1,62.

## Patentansprüche

1. Verfahren zur kontinuierlichen Herstellung von Nitrochlorbenzol durch Umsetzung von Chlorbenzol mit Schwefelsäure, Phosphorsäure, Salpetersäure und Wasser, **dadurch gekennzeichnet, dass**
a) die Einsatzstoffe Chlorbenzol, Schwefelsäure, Phosphorsäure, Salpetersäure und Wasser gleichzeitig oder in beliebiger Reihenfolge sukzessive in einen mit Mischungsorganen ausgestatteten Reaktor eingetragen werden und so vermischt werden, dass die Temperatur des Reaktionsgemisches bei der Erstvermischung 10 bis 80°C beträgt,
b) der Gehalt an Phosphorsäure bezogen auf reine H₃PO₄ im Reaktionsgemisch bei der Vermischung, bezogen auf die Summe aus Schwefelsäure, Phosphorsäure, Salpetersäure und Wasser 10 bis 50 Gew.-% beträgt,
c) die Reaktion unter adiabatischen Bedingungen abläuft,
d) am Reaktorausgang das rohe Nitrochlorbenzol von der Abfallsäure abgetrennt wird,
e) die Abfallsäure aufkonzentriert und wieder in die Nitrierreaktion zurückgeführt wird.

2. Verfahren nach Anspruch 1, **dadurch gekennzeichnet, dass** es in Gegenwart von Siliziumverbindungen durchgeführt wird.

3. Verfahren gemäß einem oder mehreren der Ansprüche 1 bis 2, **dadurch gekennzeichnet, dass** der Gehalt an Schwefelsäure bezogen auf reine H₂SO₄ im Reaktionsgemisch bei der Vermischung 30 bis 80 Gew.-% beträgt.

4. Verfahren gemäß einem oder mehreren der Ansprüche 1 bis 3, **dadurch gekennzeichnet, dass** der Gehalt an Salpetersäure, bezogen auf reine HNO₃ im Reaktionsgemisch bei der Vermischung, bezogen auf die Summe aus Schwefelsäure, Salpetersäure und Wasser 3 bis 10 Gew.-% beträgt.

5. Verfahren gemäß einem oder mehreren der Ansprüche 1 bis 4, **dadurch gekennzeichnet, dass** Salpetersäure in Form einer 60 bis 70 %igen Salpetersäure eingesetzt wird.

6. Verfahren gemäß einem oder mehreren der Ansprüche 1 bis 5, **dadurch gekennzeichnet, dass** der Gehalt an Wasser im Reaktionsgemisch bei der Vermischung, bezogen auf die Summe von Schwefelsäure bezogen auf reine H₂SO₄, Phosphorsäure bezogen auf reine H₃PO₄, Salpetersäure bezogen auf reine HNO₃ und Wasser, 5 bis 30 Gew.-% beträgt.

7. Verfahren gemäß einem oder mehreren der Ansprüche 1 bis 6, **dadurch gekennzeichnet**, das Chlorbenzol in einer Menge von 1 bis 1,3 Mol pro Mol Salpetersäure eingesetzt wird.

8. Verfahren gemäß einem oder mehreren der Ansprüche 1 bis 7, **dadurch gekennzeichnet, dass** das eingesetzte Chlorbenzol Nitrochlorbenzol in einer Menge von 0 bis 20 Gew.-% enthält.

9. Verfahren gemäß einem oder mehreren der vorangegangenen Ansprüche 1 bis 8, **dadurch gekennzeichnet, dass** die mittlere Mischleistungsdichte 1,5 bis 40 Watt/l beträgt.

10. Verfahren gemäß einem oder mehreren der Ansprüche 1 bis 9, **dadurch gekennzeichnet, dass** die Temperatur des Reaktionsgemisches bei der Erstvermischung 20 bis 50°C beträgt.

11. Verfahren gemäß einem oder mehreren der Ansprüche 1 bis 10, **dadurch gekennzeichnet, dass** die Aufkonzentrierung der Abfallsäure in einem Verdampfer durchgeführt wird.

12. Verfahren gemäß Anspruch 11, **dadurch gekennzeichnet, dass** die Aufkonzentrierung der Abfallsäure in einem Verdampfer bei einem Druck von 60 bis 200 mbar durchgeführt wird.

13. Verfahren gemäß einem oder mehreren der Ansprüche 11 bis 12, **dadurch gekennzeichnet, dass** die Temperatur der Abfallsäure im Austritt des Verdampfers 100 bis 200°C beträgt.

14. Verfahren gemäß einem oder mehreren der Ansprüche 11 bis 13, **dadurch gekennzeichnet, dass** es sich bei dem Verdampfer um einen kaskadierten Verdampfer mit Tantalrohrbündel handelt.
